# EUROPEAN PATENT APPLICATION

(11) **EP 4 026 587 A1**
(43) Date of publication of application: **13.07.2022**
(21) Application number: 20871254.7
(22) Date of filing: 30.09.2020
(51) Int. Cl.: A61P 9/00, C12N 5/077, A61K 35/34

(54) **METHOD FOR INCREASING PROPORTION OF CD56+ CELLS**

(30) Priority: 30.09.2019 JP 2019179003
(71) Applicant: TERUMO Kabushiki Kaisha, Tokyo 151-0072 (JP)
(72) Inventor: YUKI, Risa, Ashigarakami-gun, Kanagawa 259-0151 (JP)
(74) Representative: Prüfer & Partner mbB Patentanwälte · Rechtsanwälte
(86) International application number: PCT/JP2020/037093
(87) International publication number: WO 2021/065989

(57) **Abstract**

It was an object to provide a method for increasing a proportion of CD56 positive cells in sampled skeletal muscle tissue. The above problem was solved by a method for increasing a proportion of CD56 positive cells in skeletal muscle tissue, including a step of soaking the sampled skeletal muscle tissue in a preservation solution.

## Description

### Technical Field

The present invention relates to a method for increasing a proportion of CD56 positive cells in skeletal muscle tissue, a method for producing a cell population with a high proportion of CD56 positive cells, a method for producing a graft using them, a graft produced by the production method, and the like.

### Background Art

Despite recent innovative advances in treatment for heart disease, a treatment system for severe heart failure has not yet been established. It is considered that development of new regenerative medicine is indispensable as a solution for treatment of severe heart failure. In severe myocardial infarction and the like, cardiomyocytes become dysfunctional, and furthermore, proliferation of fibroblast cells and fibrosis of stroma progress and heart failure occurs. With the progress of heart failure, cardiomyocytes are damaged and fall into apoptosis, but since cardiomyocytes hardly undergo cell division, the number of cardiomyocytes decreases and deterioration of cardiac function further progresses. A cell transplantation method is considered to be useful for cardiac function recovery for such a severe heart failure patient, and clinical application using myoblast cells has already been started.

Recently, as an example thereof, a three-dimensionally constructed cell culture that can be transplanted into a heart containing myoblast cells and a method for producing the cell culture have been provided (Patent Literature 1). Myoblast cells used for such cell transplantation are usually obtained by separating CD56 positive cells such as myoblast cells and muscle satellite cells from skeletal muscle tissue to be transplanted, and as a measure for increasing a ratio of CD56 positive cells, for example, a method of discarding an enzyme treatment solution obtained by performing enzyme treatment by soaking skeletal muscle tissue in a protease solution for a predetermined time, and then collecting cells contained in an enzyme treatment solution obtained by performing enzyme treatment by soaking skeletal muscle tissue again in a protease solution for a predetermined time (Patent Literature 2), and use of a culture medium containing serum and a basal medium as a culture solution for culturing only muscle cells, with a serum concentration of 10 to 40 vol% in the total culture solution and a basal medium of 50 to 90 vol% in the total culture solution, and substantially free of glucose (Patent Literature 3) have been known.

### Citation List

### Patent Literature

Patent Literature 1: JP 2007-528755 A
Patent Literature 2: JP 2011-110368 A
Patent Literature 3: JP 2018-000194 A

### Summary of Invention

### Technical Problem

Since CD56 positive cells such as myoblast cells and muscle satellite cells are present in a deep part of muscle fibers constituting skeletal muscle, that is, between basement membrane (basal membrane) and plasma membrane, it is necessary to destroy the entire skeletal muscle tissue in order to separate CD56 positive cells from a sampled skeletal muscle tissue, and as a result, a cell population obtained by separation inevitably contains many non-CD56 positive cells such as fibroblast cells constituting the skeletal muscle tissue. A method for easily and stably increasing a proportion of CD56 positive cells in order to produce higher quality grafts is desired.

### Solution to Problem

As a result of intensive studies to solve the above-described problems, the present inventor has found that the proportion of CD56 positive cells in skeletal muscle tissue is dramatically increased when the sampled skeletal muscle tissue is soaked in a preservation solution and maintained, and further advanced research, thereby completing the present invention.

That is, the present invention relates to the following.
[1] A method for increasing a proportion of CD56 positive cells in skeletal muscle tissue, including a step of soaking the sampled skeletal muscle tissue in a preservation solution.
[2] The method according to [1], in which the soaking step includes soaking the skeletal muscle tissue until the proportion of non-CD56 positive cells in the sampled skeletal muscle tissue is reduced to 20% or less.
[3] The method according to [1] or [2], in which the soaking step includes soaking the skeletal muscle tissue in the preservation solution until the degree of swelling of the skeletal muscle tissue becomes 1.2 or more.
[4] The method according to any one of [1] to [3], in which the soaking step includes soaking in the preservation solution for 12 hours or more.
[5] A method for producing a cell population with a high proportion of the number of CD56 positive cells, including:
   the method according to any one of [1] to [4].
[6] The method according to [5], further including a step of separating the cell population from the skeletal muscle tissue.
[7] A cell population with a high proportion of CD56 positive cells obtained by the method according to [5] or [6] .
[8] A method for producing a graft using the cell population according to [7].
[9] A graft obtained by the method according to [8].
[10] A method for treating heart disease in a subject in need thereof, including administering to the subject a therapeutically effective amount of the cell population according to [7] or the graft according to [9].

### Advantageous Effects of Invention

The proportion of CD56 positive cells in the sampled tissue can be increased, whereby it is possible to easily and stably provide a high-quality cell population, graft, and the like.

### Brief Description of Drawings

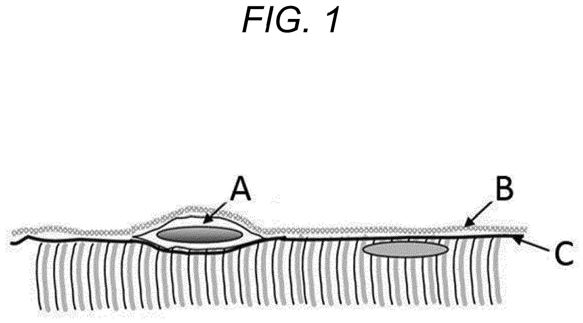

Fig. 1 is a schematic view showing that a muscle satellite cell (A) exists between a basement membrane (basal membrane) (B) and a plasma membrane (C) of muscle fibers constituting skeletal muscle.

### Description of Embodiments

Unless otherwise defined herein, all technical and scientific terms used herein have the same meaning as commonly understood by those skilled in the art. All patents, applications, published applications, and other publications referenced herein are hereby incorporated by reference in their entirety. Hereinafter, the present invention will be described based on preferred embodiments of the present invention.

### <Method for increasing proportion of CD56 positive cells in tissue>

The present invention includes a method for increasing a proportion of CD56 positive cells in skeletal muscle tissue, including a step of soaking the sampled skeletal muscle tissue in a preservation solution.

The "CD56 positive cell" in the present disclosure refers to a cell characterized by cell surface marker CD56. The CD56 positive cell may be a somatic cell, an undifferentiated stem cell (for example, a pluripotent stem cell such as a myoblast cell or a tissue stem cell such as a cardiac stem cell, a mesenchymal stem cell, or the like), or the like, but the CD56 positive cell contained in the skeletal muscle tissue is typically a skeletal muscle precursor cell such as a myoblast cell and a muscle satellite cell.

The "proportion of CD56 positive cells" in the present disclosure refers to a ratio of the number of CD56 positive cells to the total number of cells constituting a tissue or cell population containing CD56 positive cells and cells other than CD56 positive cells (also referred to as non-CD56 positive cells).

A cell population separated from the skeletal muscle tissue is mainly composed of, for example, skeletal muscle precursor cells such as myoblast cells and muscle satellite cells, and fibroblast cells. In this case, the skeletal muscle precursor cells are CD56 positive cells, and an "increase in the proportion of CD56 positive cells" means to increase the proportion of skeletal muscle precursor cells. Thus, in one embodiment of the present invention, the CD56 positive cells are myoblast cells and/or muscle satellite cells and the non-CD56 positive cells are fibroblast cells.

Examples of a method for measuring the proportion of CD56 positive cells include labeling with an anti-CD56 antibody, and dividing the number of positive cells bound to the antibody by the total number of cells counted. The counting of CD56 positive cells can be performed by microscopic observation of a specimen stained with a specific antibody, image analysis of a microscopic image, flow cytometry analysis of a cell population stained with a specific antibody, or the like.

When the CD56 positive cell is, for example, a myoblast cell, it can be identified by, in addition to CD56, for example, markers such as, but not limited to, Pax7, GATA4, MyoD, α7 integrin, myosin heavy chain IIa, myosin heavy chain IIb, myosin heavy chain IId (IIx), Myf5, and myogenin. When the CD56 positive cell is a muscle satellite cell, it can be identified by, in addition to CD56, for example, markers such as, but not limited to, Pax7, α7 integrin, CD34, CXCR4, and CD29.

The skeletal muscle tissue (hereinafter also referred to as tissue) used in the present invention may be derived from any organism. Examples of the organism include, but not limited to, humans, non-human primates, rodents (mice, rats, hamsters, guinea pigs, and the like), dogs, cats, pigs, horses, cows, goats, sheep, and the like. The tissue used in the present invention may be sampled from a living body, for example, a recipient. Therefore, the method for increasing a proportion of CD56 positive cells of the present invention may include a step of sampling skeletal muscle tissue from a living body or the like and/or a step of washing the sampled skeletal muscle tissue, before soaking.

The preservation solution used in the present invention is not particularly limited as long as the CD56 positive cells in the tissue can survive, and examples thereof include physiological saline, various physiological buffers (for example, PBS, HBSS, and the like), cell culture solutions based on various basal media for cell culture, tissue preservation solutions or organ preservation solutions, and the like. Examples of the basal medium include, but not limited to, DMEM, MEM, F12, DME, RPMI1640, MCDB (MCDB102, 104, 107, 120, 131, 153, 199, and the like), L15, SkBM, RITC80-7, DMEM/F12, and the like. Many of these cell culture solutions, tissue preservation solutions and organ preservation solutions are commercially available, and compositions thereof are also known. The basal medium may be used with standard composition (for example, in the commercially distributed state), or composition of, for example, saccharides, amino acids, vitamins, electrolytes, or the like may be appropriately changed according to the cell type and cell conditions. Examples of the known tissue preservation solution include, but are not limited to, a tissue preservation solution obtained by adding glucose to HBSS, AQIXR RS-I (AQIX LTD, Cat No: RSIKIT4), ThelioKeep (BioVerde, Cat No: TPO-A1), and the like. Examples of the organ preservation solution include, but are not limited to, Euro-Collins solution, UW solution, HTK solution, Celsior solution, ETKyoto solution, IGL-1 solution, EP-TU solution, and the like. In addition to normal serum (for example, bovine serum such as fetal bovine serum (FBS), horse serum, human serum, and the like) and various growth factors (for example, FGF, EGF, VEGF, HGF, and the like), any additives such as Nrf2 activators and antibiotics may be contained. A HBSS-based tissue preservation solution or AQIXR RS-I is preferred.

The present inventor has found that the proportion of CD56 positive cells in skeletal muscle tissue can be increased by soaking the sampled skeletal muscle tissue in a preservation solution. An increase in the proportion of CD56 positive cells in skeletal muscle tissue is caused by a reduction in non-CD56 positive cells in the skeletal muscle tissue by soaking. Therefore, in one embodiment of the present invention, the step of soaking may be a step of reducing non-CD56 positive cells by soaking.

Usually, when the sampled skeletal muscle tissue is soaked in a tissue preservation solution, the total number of cells in the tissue decreases with time. However, in skeletal muscle tissue, CD56 positive cells such as myoblast cells and muscle satellite cells are localized in a deep portion of muscle fibers constituting skeletal muscle tissue, that is, in a special environment sandwiched between a basement membrane and a plasma membrane. For this reason, even if the soaking of the skeletal muscle tissue is maintained, conditions essential for survival of CD56 positive cells such as osmotic pressure, nutritional state, and scaffold structure of the environment are likely to be maintained, and it is considered that the CD56 positive cells are relatively maintained while the non-CD56 cells such as fibroblast cells are reduced. As described above, as long as the CD56 positive cells are present in the skeletal muscle tissue, they have a survival advantage over the non-CD56 positive cells, and thus it can be understood by those skilled in the art that the reduction in non-CD56 positive cells due to soaking contributes to the increase in the proportion of CD56 positive cells.

Therefore, the step of soaking in the preservation solution of the present invention is performed before the step of separating a cell population from the skeletal muscle tissue, and is typically performed on skeletal muscle tissue not subjected to enzyme treatment (enzyme digestion), but may be performed, for example, after a step of crushing and/or shredding (mincing) as long as the tissue structure of the skeletal muscle tissue is maintained.

The step of soaking in the preservation solution of the present invention reduces the non-CD56 positive cells in skeletal muscle tissue, and is different from simple washing and preservation. On the contrary, the soaking of the present invention may be performed as washing of tissues or preservation as long as the non-CD56 positive cells can be reduced.

In the present invention, the number of CD56 positive cells in skeletal muscle tissue does not necessarily need to be maintained during soaking, and those skilled in the art can understand that when the reduction rate of CD56 positive cells is lower than the reduction rate of non-CD56 positive cells, it contributes to an increase in the proportion of CD56 positive cells.

The soaking of the present invention may be performed with a single preservation solution or with a plurality of preservation solutions. The soaking of the present invention is preferably performed until the reduction in non-CD56 positive cells in the tissue is stopped.

In one embodiment of the present invention, the soaking can be performed until the ratio of non-CD56 positive cells in skeletal muscle tissue decreases to, for example, 20% or less, 19% or less, 18% or less, 17% or less, 16% or less, 15% or less, 14% or less, 13% or less, 12% or less, 10% or less, 9% or less, 8% or less, 7% or less, 6% or less, 5% or less, 4% or less, 3% or less, 2% or less, 1% or less, 0%, preferably 15% or less, more preferably 10% or less, and further preferably 5% or less.

Therefore, in one embodiment of the present invention, the soaking can be performed until the ratio of CD56 positive cells in skeletal muscle tissue is, for example, but are not limited to, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, 99% or more, 100%, preferably 85% or more, more preferably 90% or more, and further preferably 95% or more.

In another aspect of the present invention, the soaking is not limited as long as the ratio of non-CD56 positive cells in skeletal muscle tissue decreases by, for example, but are not limited to, 10% or more, 20% or more, 30% or more, 40% or more, 50% or more, 60% or more, 70% or more, 80% or more, 90% or more, or 95% or more, as compared with the tissue before soaking.

In another aspect of the present invention, the soaking is not limited as long as the ratio of CD56 positive cells in skeletal muscle tissue increases by 1% or more, 2% or more, 3% or more, 4% or more, 5% or more, 6% or more, 7% or more, 8% or more, 9% or more, 10% or more, 11% or more, 12% or more, 13% or more, 14% or more, 15% or more, 16% or more, 17% or more, 18% or more, 19% or more, or 20% or more, as compared with that before soaking.

The ratio of CD56 positive cells in skeletal muscle tissue may be determined by microscopic observation of a specimen obtained by staining the CD56 positive cells in skeletal muscle tissue with a specific antibody, image analysis of a microscopic image, or may be determined by flow cytometry analysis of a cell population stained with a specific antibody after separating the cell population by a known method, or the like.

In the present disclosure, the "swelling" of skeletal muscle tissue refers to an increase in weight of skeletal muscle tissue due to the soaking of the present invention. When the skeletal muscle tissue is soaked in the preservation solution, the amount of the preservation solution contained in the skeletal muscle tissue increases over time, whereby the weight of the tissue after soaking increases. In the present invention, the "degree of swelling" is a value obtained by dividing the weight of tissue before soaking by the weight of tissue after soaking.

In one embodiment of the present invention, the soaking can be performed until the degree of swelling of skeletal muscle tissue becomes, for example, 1.05 or more, 1.1 or more, 1.2 or more, 1.3 or more, 1.4 or more, 1.5 or more, 1.6 or more, 1.7 or more, 1.8 or more, 1.9 or more, 2.0 or more, preferably 1.2 or more, and further preferably 1.5 or more.

In one embodiment of the present invention, the soaking time is not particularly limited as long as the CD56 positive cells can survive, and the lower limit value is, for example, 1 hour or more, 2 hours or more, 4 hours or more, 5 hours or more, 6 hours or more, 8 hours or more, 10 hours or more, or 12 hours or more. From the viewpoint of sufficiently reducing the non-CD56 positive cells, the soaking time is preferably 12 hours or more, 24 hours or more, 48 hours or more, 49 hours or more, 60 hours or more, 72 hours or more, 84 hours or more, 96 hours or more, 180 hours or more, 240 hours, 350 hours or more, and may be 750 hours, 1500 hours or more, or 3000 hours or more.

In one embodiment of the present invention, the lower limit value of the soaking time is 2500 hours or less, 1500 hours or less, 750 hours or less, 672 hours or less, 350 hours or less, 336 hours or less, 240 hours or less, 180 hours or less, 170 hours or less, or the like, and the upper limit value and the lower limit value described above can be arbitrarily selected.

In one embodiment of the present invention, the combination of the upper limit and the lower limit of the soaking time can be selected from the following. For example, it can be 5 hours to 672 hours, 5 hours to 336 hours, 6 hours to 336 hours, 12 hours to 336 hours, 12 hours to 170 hours, and 49 hours to 170 hours.

The soaking temperature is not particularly limited as long as the survival of CD56 positive cells can be maintained, and is 1 to 40°C, 2 to 20°C, 3 to 15°C, 2 to 10°C, 2 to 8°C, 4 to 6°C, preferably 1 to 15°C, and further preferably 2 to 8°C. The temperature in the step of soaking may be kept constant or varied. The soaking may be performed by using a known container and leaving the container, or may be performed by shaking or turning the container.

### <Method for producing cell population having high proportion of number of CD56 positive cells>

Another aspect of the present invention is a method for producing a cell population, including the method for increasing a proportion of the number of CD56 positive cells. The production method of the present invention includes a step of separating the cell population from skeletal muscle tissue having an increased proportion of CD56 positive cells by the method of the present invention by a known method.

The step of separating is not particularly limited as long as the cell population can be separated, and can include, for example, a step of crushing and/or shredding (mincing) skeletal muscle tissue and/or subjecting the skeletal muscle tissue to enzyme treatment. The step of crushing and/or shredding and/or subjecting the skeletal muscle tissue to enzymatic treatment can be carried out using any known method. The method for producing a cell population of the present invention may further include a step of culturing the separated cell population and a step of passaging the cultured cells.

Cell culture and passaging (subculture) can be performed using any known method.

The method for producing a cell population of the present invention may further include a step of introducing a gene into collected cells. The gene to be introduced is not particularly limited as long as it is useful for treatment of a disease to be treated, and may be, for example, a cytokine such as HGF. The gene can be introduced using any known method such as a calcium phosphate method, a lipofection method, an ultrasonic introduction method, an electroporation method, a particle gun method, a method using a viral vector such as an adenoviral vector or a retroviral vector, or a microinjection method.

### <Cell population>

Next, the cell population of the present invention will be described.

The cell population of the present invention is obtained by the method for producing a cell population of the present invention, and has a high ratio of CD56 positive cells. The height of the ratio of CD56 positive cells in the cell population is, for example, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, 99% or more, 100%, preferably 85% or more, more preferably 90% or more, and further preferably 95% or more.

Since the cell population of the present invention contains a high proportion of CD56 positive cells, it is extremely useful as a supply source of skeletal muscle precursor cells such as myoblast cells and/or muscle satellite cells used for heart disease treatment and the like. The cell population of the present invention is preferably sterile. Further, the cell population of the present invention may be adhered to a culture vessel, suspended in an arbitrary liquid, or cryopreserved.

### <Method for producing graft>

Another aspect of the present invention relates to a method for producing a graft using a cell population with a high proportion of CD56 positive cells obtained by the method of the present invention.

In the present invention, the "graft" means a structure for transplantation into a living body, and particularly means a transplantation structure containing a cell as a component. In a preferred embodiment, the graft is a transplantation structure that does not contain cells and structures other than cell-derived substances (for example, scaffold, and the like). Examples of the graft in the present disclosure include, but are not limited to, a sheet-shaped cell culture, a spheroid, a cell aggregate (cell mass), and the like, preferably a sheet-shaped cell culture or a spheroid, and more preferably a sheet-shaped cell culture.

In the present disclosure, the "sheet-shaped cell culture" refers to a sheet shape formed with cells connected to each other. In the present disclosure, the "spheroid" refers to a nearly spherical shape formed with cells connected to each other The cells may be connected to each other directly (including via a cellular element such as an adhesion molecule) and/or via a mediator. The mediator is not particularly limited as long as it is a substance which can connect cells at least physically (mechanically), and examples thereof include an extracellular matrix and the like. The mediator is preferably derived from cells, particularly derived from cells constituting the sheet-shaped cell culture. Although the cells are connected at least physically (mechanically), the cells may further be connected functionally, for example, chemically or electrically. The sheet-shaped cell culture may be composed of one cell layer (monolayer) or composed of two or more cell layers (laminate (multilayer), for example, 2 layers, 3 layers, 4 layers, 5 layers, 6 layers, and the like). In addition, the sheet-shaped cell culture may have a three-dimensional structure having a thickness exceeding the thickness of one cell without the cells exhibiting a clear layer structure. For example, in a vertical cross-section of the sheet-shaped cell culture, cells may be present in a non-uniform (for example, in a mosaic manner) arrangement without being uniformly aligned in the horizontal direction.

The graft of the present disclosure, in particular, the sheet-shaped cell culture, preferably does not contain any scaffold (support). A scaffold may be used in the art in order to adhere cells on the surface thereof and/or inside thereof and maintain physical unity of the graft such as the sheet-shaped cell culture, and for example, a membrane made of polyvinylidene difluoride (PVDF) and the like are known. The graft of the disclosure can maintain its physical unity without the scaffold. In addition, the graft of the disclosure preferably consists of only substances derived from the cells constituting the graft and does not contain any other substances.

The culture substrate is not particularly limited as long as cells can form a cell culture on the culture substrate, and includes, for example, containers of various materials and/or shapes, solid or semi-solid surfaces in the containers, and the like. The structure and material of the container preferably do not allow permeation of a liquid such as a culture solution. Examples of the material include, but are not limited to, polyethylene, polypropylene, Teflon (registered trademark), polyethylene terephthalate, polymethyl methacrylate, nylon 6,6, polyvinyl alcohol, cellulose, silicon, polystyrene, glass, polyacrylamide, polydimethylacrylamide, metals (for example, iron, stainless steel, aluminum, copper, brass), and the like. Further, the container preferably has at least one flat surface. Examples of the container include, but are not limited to, a culture vessel having a bottom surface composed of a culture substrate capable of forming a cell culture and a liquid-impermeable side surface. Specific examples of the culture vessel include, but not limited to, a cell culture dish, a cell culture bottle, and the like. The bottom surface of the container may be transparent or opaque. When the bottom surface of the container is transparent, observation, counting, and the like of cells from the back side of the container are possible. In addition, the container may have a solid or semi-solid surface in its inside. Examples of the solid surface include a plate, a container, and the like of the various materials described above, and examples of the semi-solid surface include a gel, a soft polymer matrix, and the like. The culture substrate may be prepared using the above materials, or a commercially available culture substrate may be used.

Examples of preferred culture substrates include, but are not limited to, a substrate having an adhesive surface suitable for formation of a sheet-shaped cell culture, a substrate having a low adhesive surface and/or a substrate having a uniform well structure, suitable for formation of a spheroid, and the like. In the case of forming a sheet-shaped cell culture, specific examples include a substrate with a surface coated with a hydrophilic compound such as polystyrene, collagen gel, or a hydrophilic polymer subjected to corona discharge treatment, a substrate with a surface coated with an extracellular matrix such as collagen, fibronectin, laminin, vitronectin, proteoglycan, or glycosaminoglycan, a cell adhesion factor such as cadherin family, selectin family, or integrin family, and the like. Further, the above substrates are commercially available (for example, Corning(R) TC-Treated Culture Dish, Corning, and the like). In the case of forming a spheroid, examples include soft agar, a substrate with a surface coated with a non-cell adhesive compound such as a temperature-responsive gel obtained by crosslinking poly(N-isopropylacrylamide) (PIPAAm) with polyethylene glycol (PEG) (commercial name: mebiol gel), polyhydroxyethyl methacrylate (polyHEMA), or a hydrogel such as a 2-methacryloyloxyethyl phosphorylcholine (MPC) polymer, and/or a substrate having a uniform irregular structure on the surface, and the like. The above substrates are also commercially available (for example, EZSPHERE(R) and the like). The culture substrate may be transparent or opaque in whole or in part.

The surface of the culture substrate may be covered with a material whose physical properties change in response to a stimulus, for example, temperature or light. As the material, for example, a known material such as a temperature-responsive material made of a homopolymer or copolymer of a (meth)acrylamide compound, an N-alkylsubstituted (meth)acrylamide derivative (for example, N-ethylacrylamide, N-n-propylacrylamide, N-n-propylmethacrylamide, N-isopropylacrylamide, N-isopropylmethacrylamide, N-cyclopropylacrylamide, N-cyclopropylmethacrylamide, N-ethoxyethylacrylamide, N-ethoxyethylmethacrylamide, N-tetrahydrofurfurylacrylamide, N-tetrahydrofurfurylmethacrylamide, or the like), an N,N-dialkyl-substituted (meth)acrylamide derivative (for example, N,N-dimethyl(meth)acrylamide, N,N-ethylmethylacrylamide, N,N-diethylacrylamide, or the like), a (meth)acrylamide derivative having a cyclic group (for example, 1-(1-oxo-2-propenyl)-pyrrolidine, 1-(1-oxo-2-propenyl)-piperidine, 4-(1-oxo-2-propenyl)-morpholine, 1-(1-oxo-2-methyl-2-propenyl)-pyrrolidine, 1-(1-oxo-2-methyl-2-propenyl)-piperidine, 4-(1-oxo-2-methyl-2-propenyl)-morpholine, or the like), or a vinyl ether derivative (for example, methyl vinyl ether), a light-absorbing polymer having an azobenzene group, a copolymer of a vinyl derivative of triphenylmethane leukohydroxide and an acrylamide monomer, or an N-isopropylacrylamide gel containing spirobenzopyran (see, for example, JP 2-211865 A and JP 2003-33177 A) can be used without restriction. By giving a predetermined stimulus to these materials, the physical properties, for example, hydrophilicity and hydrophobicity can be changed to promote peeling of a cell culture adhered on the materials. Culture dishes covered with a temperature-responsive material are commercially available (for example, UpCell(R) of CellSeed Inc.), and these can be used in the production method of the present disclosure.

The culture substrate may have various shapes. In addition, the area is not particularly limited, and may be, for example, about 1 cm² to about 200 cm², about 2 cm² to about 100 cm², about 3 cm² to about 50 cm², or the like. Examples of the culture substrate include a circular culture dish with a diameter of 10 cm. In this case, the area is 56.7 cm². The culture surface may be flat or may have an irregular structure. In the case of having an irregular structure, it is preferable to have a uniform irregular structure.

The culture substrate may be coated (covered or coated) with serum. By using a culture substrate coated with serum, a sheet-shaped cell culture with a higher density can be formed. The phrase "coated with serum" means a state in which a serum component is adhered to the surface of the culture substrate. The state can be obtained, for example, but not limited to, by treating the culture substrate with serum. The treatment with serum includes bringing serum into contact with the culture substrate and incubating the serum for a predetermined period of time as necessary.

As the serum, heterologous serum and/or homologous serum can be used. Heterologous serum means serum derived from an organism of a different species from its recipient when a graft, particularly a sheet-shaped cell culture, is used for transplantation. For example, when the recipient is a human, serum derived from bovine or horse, for example, fetal bovine/calf serum (FBS, FCS), calf serum (CS), horse serum (HS), and the like correspond to heterologous serum. In addition, the "homologous serum" means serum derived from an organism of the same species as the recipient. For example, when the recipient is a human, human serum corresponds to the homologous serum. The homologous serum includes autologous serum (also referred to as autoserum), that is, serum derived from the recipient and allogeneic serum derived from an allogeneic individual other than the recipient. In the present specification, serum other than autologous serum, that is, heterologous serum and allogeneic serum may be collectively referred to as non-autologous serum.

The serum for coating the culture substrate is commercially available or can be prepared from blood sampled from a desired organism by a conventional method. Specific examples include a method of leaving the sampled blood at room temperature for about 20 minutes to about 60 minutes to be coagulated, centrifuging the coagulated blood at about 1000 × g to about 1200 × g, and sampling a supernatant, and the like,

When serum is incubated on the culture substrate, the serum may be used as a stock solution, or diluted serum may be used. The serum can be diluted using any medium, for example, but not limited to, water, physiological saline, various buffers (for example, PBS, HBSS, and the like), various liquid media (for example, DMEM, MEM, F12, DMEM/F12, DME, RPMI1640, MCDB (MCDB102, 104, 107, 120, 131, 153, 199, and the like), L15, SkBM, RITC80-7, and the like), and the like. The dilution concentration is not particularly limited as long as the serum component can adhere to the culture substrate, and is, for example, about 0.5% to about 100% (v/v), preferably about 1% to about 60% (v/v), and more preferably about 5% to about 40% (v/v).

The incubation time is also not particularly limited as long as the serum component can adhere to the culture substrate, and is, for example, about 1 hour to about 72 hours, preferably about 2 hours to about 48 hours, more preferably about 2 hours to about 24 hours, and further preferably about 2 hours to about 12 hours. The incubation temperature is also not particularly limited as long as the serum component can adhere to the culture substrate, and is, for example, about 0°C to about 60°C, preferably about 4°C to about 45°C, and more preferably room temperature to about 40°C.

The serum may be discarded after the incubation. As a method of discarding the serum, a conventional method of discarding a liquid such as suction with a pipette or decantation can be used. In a preferred embodiment of the present disclosure, the culture substrate may be washed with a serum-free washing solution after discarding the serum. The serum-free washing solution is not particularly restricted as long as it is a liquid medium that does not contain serum and does not adversely affect the serum component adhered to the culture substrate, and washing can be performed by, for example, but not limited to, water, physiological saline, various buffers (for example, PBS, HBSS, and the like), various liquid media (for example, DMEM, MEM, F12, DMEM/F12, DME, RPMI1640, MCDB (MCDB102, 104, 107, 120, 131, 153, 199, and the like), L15, SkBM, RITC80-7, and the like), and the like. As the washing method, a conventional method of washing a culture substrate, for example, a method of adding a serum-free washing solution to the culture substrate, stirring the solution for a predetermined time (for example, about 5 seconds to about 60 seconds), and then discarding the solution or the like can be used without restriction.

In the present disclosure, the culture substrate may be coated with a growth factor. Here, the "growth factor" means any substance that promotes proliferation of cells as compared with the case where there is no growth factor, and examples thereof include epidermal growth factor (EGF), vascular endothelial growth factor (VEGF), fibroblast growth factor (FGF), and the like. The method for coating the culture substrate with a growth factor, the discarding method, and the washing method are basically the same as those for the serum, except that the dilution concentration during the incubation is, for example, about 0.0001 µg/mL to about 1 µg/mL, preferably about 0.0005 µg/mL to about 0.05 µg/mL, and more preferably about 0.001 µg/mL to about 0.01 µg/mL.

In the present disclosure, the culture substrate may be coated with a steroid. Here, the "steroid" refers to a compound having a steroid nucleus, which may have adverse effects such as adrenocortical insufficiency and Cushing's syndrome on a living body. Examples of the compound include, but not limited to, cortisol, prednisolone, triamcinolone, dexamethasone, betamethasone, and the like. The method for coating the culture substrate with a steroid, the discarding method, and the washing method are basically the same as those for the serum, except that the dilution concentration during the incubation is, for example, about 0.1 µg/mL to about 100 µg/mL, preferably about 0.4 µg/mL to about 40 µg/mL, and more preferably about 1 µg/mL to about 10 µg/mL, as dexamethasone.

The culture substrate may be coated with any one of serum, a growth factor, and a steroid, or may be coated with any combination thereof, that is, a combination of serum and a growth factor, a combination of serum and a steroid, a combination of serum, a growth factor, and a steroid, or a combination of a growth factor and a steroid. When the culture substrate is coated with a plurality of components, the culture substrate may be coated simultaneously with a mixture of the components or coated in separate steps.

Seeding of the cell population on the culture substrate can be performed by any known method and conditions. The seeding of the cell population on the culture substrate may be performed, for example, by injecting a cell suspension obtained by suspending the cell population in a culture solution into the culture substrate (culture vessel). For injection of the cell suspension, an instrument suitable for injection operation of the cell suspension, such as a dropper or a pipette, can be used.

The culture solution used in the production method of the present invention is not particularly limited as long as the cells can be kept alive, but typically, a culture solution containing an amino acid, a vitamin, and an electrolyte as main components can be used. In one embodiment of the present invention, the culture solution is based on a basal medium for cell culture. Examples of the basal medium include, but not limited to, DMEM, MEM, F12, DMEM/F12, DME, RPMI1640, MCDB (MCDB102, 104, 107, 120, 131, 153, 199, and the like), L15, SkBM, RITC80-7, and the like. Many of these basal media are commercially available and their compositions are also known. However, when used in the production method of the present invention, the composition of the basal medium may be appropriately changed according to the cell type and culture conditions.

The cell density to be seeded is not particularly limited as long as it is a density capable of forming a sheet-shaped cell culture, but in a preferred embodiment, the cell population is seeded at a density reaching confluence or higher.

In the present disclosure, the "density reaching confluence" refers to a density at which it is assumed that when cells are seeded, the seeded cells cover the entire adhesion surface of the culture vessel without gaps. For example, the "density reaching confluence" is a density at which cells are assumed to contact each other when seeded, a density at which contact inhibition occurs, or a density at which cell proliferation is substantially stopped by contact inhibition.

Non-limiting examples of seeding densities of cell population include densities of about 7.1 × 10⁵ cells/cm² to about 3.0 × 10⁶ cells/cm², about 7.3 × 10⁵ cells/cm² to about 2.8 × 10⁶ cells/cm², about 7.5 × 10⁵ cells/cm² to about 2.5 × 10⁶ cells/cm², about 7.5 × 10⁵ cells/cm² to about 3.0 × 10⁶ cells/cm², about 7.8 × 10⁵ cells/cm² to about 2.3 × 10⁶ cells/cm², about 8.0 × 10⁵ cells/cm² to about 2.0 × 10⁶ cells/cm², about 8.5 × 10⁵ cells/cm² to about 1.8 × 10⁶ cells/cm², about 9.0 × 10⁵ cells/cm² to about 1.6 × 10⁶ cells/cm², and the like. Note that these densities are densities of all cells contained in the cell population unless otherwise specified.

In yet another embodiment, the cells can be seeded in a cell culture solution substantially free of growth factors at a density at which at least one kind of graft-forming cells that can be contained in the cell population does not substantially proliferate. In such embodiments, other cells that can be contained in the cell population can be at a density capable of proliferating while undergoing proliferation inhibition. The culture substrate used in the method of the present disclosure is as described above. In a preferred embodiment, the culture substrate may be covered with serum. In another preferred embodiment, the culture substrate may be covered with a temperature-responsive material. In a further preferred embodiment, the culture substrate may be covered with a temperature-responsive material and serum.

The cell population to be seeded includes at least one kind of CD56 positive cells, but may include two or more kinds of CD56 positive cells or may include cells other than CD56 positive cells. In one embodiment of the present disclosure, the at least one kind of CD56 positive cells contained in the cell population is preferably a myoblast cell, and more preferably a myoblast cell and a muscle satellite cell. In such embodiments, the cell population can further contain fibroblast cells. That is, examples include a graft containing myoblast cells and fibroblast cells as graft-forming cells. In yet another embodiment of the present disclosure, at least one kind of sheet-forming cells contained in the cell population is a mesenchymal stem cell. In such embodiments, the cells can further include vascular endothelial cells.

The level of proportion is as described above. The graft of the present invention may be produced by the method for producing a sheet-shaped cell culture of the present invention. The sheet-shaped cell culture of the present invention is preferably sterile. Since the graft of the present invention contains a high proportion of CD56 positive cells, the graft has a high therapeutic effect and the like as compared with a sheet-shaped cell culture not using the method of the present invention.

### <Kit>

Another aspect of the present invention relates to a kit for producing a sheet-shaped cell culture, the kit including some or all elements of the method for increasing a proportion of CD56 positive cells in skeletal muscle tissue, the method for producing a cell population with a high proportion of CD56 positive cells, and the method for producing a graft.

The kit of the present invention may contain, but not limited to, in addition to the preservation solution, cells constituting the graft (cell population containing CD56 positive cells), a culture solution, a culture dish, instruments (for example, a pipette, a dropper, a tweezer, and the like), instructions regarding a method for producing a sheet-shaped cell culture (for example, a medium in which information related to an instruction for use, a production method, or a method for collecting cryopreserved cells of the present invention is recorded, for example, a flexible disk, a CD, a DVD, a Blu-ray disk, a memory card, a USB memory, and the like), and the like.

### <Method of treating disease>

Another aspect of the present disclosure relates to a method of treating a disease in a subject in need thereof, including applying an effective amount of a cell population or graft obtained by the method of the present disclosure to the subject. In the present disclosure, the term "treatment" includes all kinds of medically acceptable preventive and/or therapeutic interventions for the purpose of cure, temporary remission, or prevention of a disease, and the like. For example, the term "treatment" includes medically acceptable interventions for various purposes including delaying or stopping of the progress of a disease associated with an abnormality of a tissue, regression or elimination of a lesion, prevention of onset of the disease, prevention of recurrence of the disease, and the like.

In the treatment method of the present disclosure, components that enhance viability, engraftment, and/or function of the graft and the like, other active ingredients useful for the treatment of the target disease, and the like can be used in combination with the graft of the present disclosure.

The treatment method of the present disclosure may include the cell population and graft of the present disclosure. The treatment method of the present disclosure may further include a step of sampling skeletal muscle tissue from a subject prior to increasing the proportion of CD56 positive cells in the skeletal muscle tissue.

In one embodiment, the subject from which the cells or the tissue serving as a source of the cells is sampled is the same individual as the subject treated by administration of the cell population, the graft, or the like.

In another embodiment, the subject from which the cells or the tissue serving as a source of the cells is sampled is a different individual of the same species as the subject treated by administration of the cell population, the graft, or the like. In another embodiment, the subject from which the cell population or the tissue serving as a source of the graft or the like is sampled is an individual of a different species from that of the subject treated by administration of the cell population, the graft, or the like.

In the present disclosure, the effective amount is, for example, an amount that can suppress the onset or recurrence of a disease, reduce symptoms, or delay or stop the progress (for example, size, weight, number, or the like of the sheet-shaped cell culture), and preferably an amount that prevents the onset and recurrence of the disease or cures the disease. Further, an amount that does not cause adverse effects exceeding benefits of administration is preferable. The amount can be appropriately determined by, for example, a test in a laboratory animal such as a mouse, a rat, a dog, or a pig, or a disease model animal, and such a test method is well known to those skilled in the art. In addition, the size of the tissue lesion to be treated may be an important index for determining the effective amount.

Examples of the administration method include intravenous administration, intramuscular administration, intraosseous administration, intrathecal administration, direct application to a tissue, and the like. The frequency of administration is typically once per treatment, but it is also possible to administer multiple times when the desired effect is not obtained. When applied to a tissue, the cell culture, composition, sheet-shaped cell culture, or the like of the present invention may be fixed to a tissue of the subject by a locking means such as a suture or a staple.

While the present invention has been described above with reference to the preferred embodiments, the present invention is not limited thereto. In the present invention, each configuration can be replaced with any one that is capable of exhibiting similar functions, or any configuration can be added.

### Examples

### Comparative Example 1: Ratio of CD56 Positive Cells of Cell Population Using Skeletal Muscle Tissue Immediately after Sampling

### [Comparative Example 1]

### [Preliminary Washing Step]

About 2 g each of skeletal muscle tissue was sampled from a pig lower limb, soaked in a tissue preservation solution ((hereinafter also referred to as a skeletal muscle rinse solution) glucose injection (Terumo Corporation) 1.6 mg/mL, gentamicin (Fuji Pharma Co., Ltd.) 0.1 mg/mL, and fungizone (Life Technologies Corporation) 2.5 µg/mL, in HBSS (Hanks' Balanced Salt Solution, Life Technologies Corporation)), washed, and immediately thereafter subjected to a separation step described later.

### [Separation Step]

### <Shredding step>

The skeletal muscle tissue washed in the preliminary washing step was placed on a petri dish with a diameter of 10 cm (Terumo Corporation). The washed skeletal muscle was then shredded (minced) in 10 mL of enzyme digestion solution (collagenase-containing solution) at room temperature. A white tissue (connective tissue) was removed therefrom.

### <Enzyme treatment step>

The enzyme digestion solution was each added according to the weight of the skeletal muscle tissue treated product to perform an enzyme reaction at 37°C. After completion of the reaction, the enzymatic digest was stirred with a conical tube (referred to as conical tube 1) and then allowed to stand, a cell strainer (BD Falcon (trademark) cell strainer, 40 µm, Japan BD) was set in a new conical tube (referred to as conical tube 2), and the collected supernatant was filtered and collected. The cell strainer was rinsed with a primary growth medium and collected in the conical tube. The collected filtrate was centrifuged, and a supernatant was discarded. A primary growth medium was added to precipitated cells to obtain a cell suspension.

### <Culture step>

The cell suspension was transferred to a culture flask (bottom area: 175 cm²), and cultured at 37°C, 5% (V/V) CO₂. After culture, the cells were collected, and the number of cells was counted.

### [Measurement of Ratio of CD56 Positive Cells]

A part of the cell suspension was reacted with a CD56 antibody, and the ratio of CD56 positive cells was measured using a flow cytometer. The ratio of CD56 positive cells refers to a proportion of CD56 positive cells in the cell population obtained by the above-described separation step. The results are shown in Table 1.

**[Table 1]**

| Sample number | 947 | 948 |
|---|---|---|
| Ratio of CD56 positive cells | 80.30% | 79.30% |

### Example 1: Change in Ratio of CD56 Positive Cells by

### Soaking Time

About 2 g each of tissue was sampled from skeletal muscle sampled from a pig lower limb different from that in the comparative example. The cells were separated by the same procedure as in Comparative Example 1, except that the soaking time in the preliminary washing step of Comparative Example 1 was changed to 17 hours, 68 hours, and 161 hours (soaking temperature 2 to 8°C).

The ratio of CD56 positive cells of the separated cells was measured in the same manner as in Comparative Example 1. In addition, the number of collected cells and viability were counted using a hemocytometer. The number of collected cells counted, the viability, and average of the ratio of CD56 positive cells are shown in Table 2.

**[Table 2]**

| | | | | |
|---|---|---|---|---|
| Skeletal muscle rinse solution | Period from sampling to preservation | 17 hour | 2 days 20 hour (68 hour) | 6 days 17 hour (161 hour) |
| | Storage: 2 to 8°C | | | |
| | Treatment amount of skeletal muscle | 2.02 g | 2.02 g | 2.03 g |
| | Enzyme | 60 min | 60 min | 60 min |
| | treatment time | | | |
| | Number of seeding | 3 | 3 | 3 |
| | Viability | 99% | 96% | 98% |
| | Number of collected cells | 1.28 × 10⁶ (cells) | 3.19 × 10⁵ (cells) | 1.12 × 10⁵ (cells) |
| | Ratio of CD56 positive cells | 94% | 95% | 91% |

It was revealed that the ratio of CD56 positive cells dramatically increased by maintaining a state in which the skeletal muscle tissue was soaked in the tissue preservation solution. In addition, since all of them showed high viability and high number of collected cells, it was suggested that non-CD56 positive cells were killed by soaking, but CD56 positive cells were maintained at a high ratio.

### Example 2: Change in Ratio of CD56 Positive Cells by Soaking Time

About 3 to 4 g each of tissue was sampled from skeletal muscle sampled from a pig lower limb different from that in Example 1. The cells were separated by the same procedure as in Comparative Example 1, except that the soaking time in the preliminary washing step of Comparative Example 1 was changed to 15 hours, 66 hours, and 140 hours (soaking temperature 2 to 8°C), and the number of collected living cells, the count of viability, and the ratio of CD56 positive cells using a hemocytometer were measured. The number of collected cells, the viability, and average of the ratio of CD56 positive cells are shown in Table 3.

**[Table 3]**

| | | | | |
|---|---|---|---|---|
| Skeletal muscle rinse solution | Period from sampling to preservation | 15 hour | 2 days 18 hour (66 hour) | 5 days 20 hour (140 hour) |
| | Storage: 2 to 8°C | | | |
| | Treatment amount of skeletal muscle | 3.78 g | 3.13 g | 3.65 g |
| | Enzyme treatment time | 60 min | 60 min | 60 min |
| | Number of seeding | 7 | 5 | 5 |
| | Viability | 95% | 97% | 97% |
| | Number of collected cells | 9.12 × 10⁵ (cells) | 7.18 × 10⁵ (cells) | 7.11 × 10⁵ (cells) |
| | Ratio of CD56 positive cells | | | 98% |

It has been revealed that the ratio of CD56 positive cells dramatically increases by soaking the skeletal muscle tissue, regardless of individual differences and the treatment amount of skeletal muscle tissue.

### Example 3: Change in Ratio of CD56 Positive Cells by Composition of Soaking Liquid

About 2.0 g each of tissue was sampled from skeletal muscle sampled from the pig lower limb of the same individual as in Example 1. The cells were separated by the same procedure as in Example 1, except that AQIXR RS-I (Cat No: RSIKIT4) was used instead of the tissue transport liquid used in the preliminary washing step in Example 1, and the number of collected cells, the count of viability, and the ratio of CD56 positive cells by a hemocytometer were measured. The number of collected cells counted, the viability, and average of the ratio of CD56 positive cells are shown in Table 4.

**[Table 4]**

| | | | | |
|---|---|---|---|---|
| AQIX | Period from sampling to preservation | 17 hour | 2 days 20 hour (68 hour) | 6 days 17 hour (161 hour) |
| | Storage: 2 to 8°C | | | |
| | Treatment amount of skeletal muscle | 1.99 g | 2.03 g | 2.03 g |
| | Enzyme treatment time | 60 min | 60 min | 60 min |
| | Number of seeding | 3 | 3 | 3 |
| | Viability | 100% | 98% | 98% |
| | Number of collected cells | 4.68 × 10⁵ (cells) | 2.73 × 10⁵ (cells) | 7.97 × 10⁵ (cells) |
| | Ratio of CD56 positive cells | 96% | 86% | 96% |

It was revealed that even when skeletal muscle tissues were soaked in soaking liquids having different compositions, the ratio of CD56 positive cells dramatically increased.

About 4 to 6 g of skeletal muscle tissue was sampled from the lower limbs of 6 human individuals. About 8 to 9 g of skeletal muscle tissue was sampled from the lower limbs of 2 pigs. Soaking was performed for 15 to 22 hours. The weight of skeletal muscle tissue at the time of sampling and after soaking (at the time of shredding) is shown in Table 5.

**[Table 5]**

| Animal species | Individual number | Skeletal muscle amount at sampling (g) | Skeletal muscle amount at shredding (g) | Degree of swelling | Soaking time (h) |
|---|---|---|---|---|---|
| Human | 1 | 5.3 | 7.82 | 1.5 | 18 |
| | 2 | 4.3 | 6.06 | 1.4 | 20 |
| | 3 | 6.4 | 9.84 | 1.5 | 20 |
| | 4 | 5.3 | 8.31 | 1.6 | 21 |
| | 5 | 4.0 | 5.07 | 1.3 | 21 |
| | 6 | 5.5 | 7.09 | 1.3 | 22 |
| Pig | 1 | 8.4 | 10.15 | 1.2 | 15 |
| | 2 | 9.1 | 10.57 | 1.2 | 19 |

From Table 5, it became clear that the tissue weight increased by 1.2 times or more as compared with that at the time of sampling by soaking.

## Claims

1. A method for increasing a proportion of CD56 positive cells in skeletal muscle tissue, comprising
soaking a sampled skeletal muscle tissue in a preservation solution.

2. The method according to claim 1, wherein the soaking step comprises soaking the skeletal muscle tissue until the proportion of non-CD56 positive cells in the sampled skeletal muscle tissue is reduced to 20% or less.

3. The method according to claim 1 or 2, wherein the soaking step comprises soaking the skeletal muscle tissue in the preservation solution until the degree of swelling of the skeletal muscle tissue becomes 1.2 or more.

4. The method according to any one of claims 1 to 3, wherein the soaking step comprises soaking in the preservation solution for 12 hours or more.

5. A method for producing a cell population with a high proportion of the number of CD56 positive cells, comprising
the method according to any one of claims 1 to 4.

6. The method according to claim 5, further comprising
separating the cell population from the skeletal muscle tissue.

7. A cell population with a high proportion of CD56 positive cells obtained by the method according to claim 5 or 6.

8. A method for producing a graft using the cell population according to claim 7.

9. A graft obtained by the method according to claim 8.

10. A method for treating heart disease in a subject in need thereof, comprising
administering to the subject a therapeutically effective amount of the cell population according to claim 7 or the graft according to claim 9.
